# EUROPEAN PATENT SPECIFICATION

(11) **EP 2 370 002 B1**
(45) Date of publication and mention of the grant of the patent: **25.07.2018**
(21) Application number: 09829467.1
(22) Date of filing: 20.11.2009
(51) Int. Cl.: A61B 17/04, A61B 17/06, A61B 17/068

(54) **APPARATUS FOR MINIMALLY INVASIVE SUTURING**
VORRICHTUNG FÜR MINIMAL-INVASIVE NAHT
APPAREIL POUR UNE SUTURE À INVASION MINIMALE

(30) Priority: 25.11.2008 US 200180 P
(43) Date of publication of application: 05.10.2011
(73) Proprietor: Endoevolution, Llc, North Chelmsford, MA 01863 (US)
(72) Inventor: MEADE, John, C., Mendon MA 01756 (US); GRIFFITHS, Jerry, R., Pembroke MA 02359 (US); DIFRANCESCO, Francis, J., Foxboro MA 02035 (US); CLARK, Richard, Holliston MA 01746 (US)
(74) Representative: Soames, Candida Jane
(86) International application number: PCT/US2009/006212
(87) International publication number: WO 2010/062380

(56) References cited:
- EP-A1- 1 839 591
- WO-A1-99/12482
- WO-A2-2007/089603
- US-A- 5 387 221
- US-A- 5 571 119
- US-A- 5 713 910
- US-A- 5 713 910
- US-A1- 2004 034 372

## Description

### CROSS-REFERENCE TO RELATED APPLICATIONS

### FIELD

The embodiments disclosed herein relate to a medical device for suturing tissue, and more particularly to a device for the manipulation and control of a suturing needle during minimally invasive suturing and methods for making such a device.

### BACKGROUND

Minimally invasive surgery (MIS) has allowed physicians to carry out many surgical procedures with less pain and disability than conventional, open surgery. Unlike conventional open surgery, where the surgical site is readily accessible through a large incision, enabling the surgeon to easily visualize and manipulate both tissue and instruments, MIS requires the surgeon to operate remotely by inserting and manipulating instruments through small punctures ("keyhole surgery") or through natural orifices, including for example the vagina, the esophagus, or the anus.

In MIS, a small puncture is typically made in the body. Medical instruments are then inserted through a cannula. A cannula has a small inside diameter, typically 5-10 millimeters (mm), and sometimes up to 20 millimeters (mm) or more. A number of such cannulas may be inserted into the body for any given operation. Minimally invasive surgical instruments are necessarily smaller, and are also generally longer and therefore are more difficult to manipulate with precision.

Perhaps the most problematic surgical task in MIS is suturing. Suturing requires coordinated manipulation with both hands of small needles and sutures that are difficult to visualize (particularly when only indirect, two-dimensional video imaging is available) as well as the several instruments (including needle-drivers and pick-up forceps) ordinarily used to suture by hand. In an environment characterized by limited space, limited visualization, and limited mobility, many surgeons find minimally invasive suturing by hand an extremely difficult, often virtually impossible, surgical task.

In the preferred method of suturing by hand, a grasping forceps ("needle driver") is held by the surgeon and is used to grip a curved needle near the needle's tail. Pronation of the surgeon's wrist drives the needle into the tissue. When the point of the curved needle emerges from the tissue, the surgeon releases the needle from the grip of the needle driver and grasps the point with another forceps ("pick-ups"). The surgeon then pulls the curved needle by the needle point, preferably in a circular path following the arc of the needle's curvature to follow the most atraumatic path through the tissue, until the entire length of the needle has exited the tissue. Each time a stitch is placed, the curved needle is thus driven around in a complete circular arc. Individual (interrupted) stitches are placed by tying off the suture following placement of each stitch. Running (continuous) stitches are placed by repeatedly driving the curved needle in a complete circular arc repeatedly until the desired length of suture and number of stitches has been placed. In order to place additional interrupted or continuous stitches, the surgeon must let go of the point of the needle and re-grasp the needle near the needle's tail.

In the manual suturing technique described above, the direct handling of the needle can result in accidental needle pricks through a surgeon or nurse's gloves, posing a potential risk of infection for the surgeon, nurse, staff, and patient, or cause the needle to become contaminated with pathogenic bacteria that can cause onset of infection at the site of the sutures. There is also a risk of the needle penetrating internal organs or vessels and causing a serious, and often fatal infection.

Various devices for suturing for MIS are described in U.S. Pat. No. 5,643,295 entitled "Methods and Apparatus for Suturing Tissue"; U.S. Pat. No. 5,665,096 entitled "Needle Driving Apparatus and Methods of Suturing Tissue"; U.S. Pat. No. 5,665,109 entitled "Methods and Apparatus for Suturing Tissue"; U.S. Pat. No. 5,759,188 entitled "Suturing Instrument with Rotatably Mounted Needle Driver and Catcher"; U.S. Pat. No. 5,860,992 entitled "Endoscopic Suturing Devices and Methods"; U.S. Pat. No. 5,954,733 entitled "Suturing Instrument with Rotatably Mounted Needle Driver and Catcher"; U.S. Pat. No. 6,719,763 entitled "Endoscopic Suturing Device"; and U.S. Pat. No. 6,755,843 entitled "Endoscopic Suturing Device".

Assignees' U.S. Pat. No. 5,437,681, U.S. Pat. No. 5,540,705 and U.S. Pat. No. 6,923,819 disclose a suturing device with thread management comprising a protective cartridge, suturing needle and needle rotation drive. The devices described in the above-mentioned patents and patent application comprise a mechanism for driving a protected needle however, the needle is rotated about an axis that is parallel to the axis of the device. In addition, the orientation and size of the suturing device makes it difficult to visualize and cumbersome to use for MIS.

Therefore, there remains a need in the art for a minimally invasive suturing device that is easily manipulated within the small diameter of the cannula; functions in an environment characterized by limited space, limited visualization, and limited mobility; mimics the preferred method of suturing used by surgeons; permits the surgeon to secure and tie knots quickly and with controlled tension; places continuous stitches; and protects user's from accidental needle sticks during needle handling, as well as internal organs and vessels, from inadvertent needle-pricks. EP1839591 A1 discloses a suturing device on which the preamble of claim 1 is based,

### Summary

Claim 1 defines the invention and the dependent claims disclose the preferred embodments.

Devices and exemplary methods for minimally invasive suturing of tissue internal to a body are disclosed herein. Accordingly, the invention provides a suturing device in accordance with the independent claim.

. According to aspects illustrated herein, there is provided a medical device for closing openings internal to a patient's body, which closely emulates or replicates the manual suturing actions carried out by a surgeon. The device offers several advantages over conventional methods used by surgeons for suturing tissue during minimally invasive surgery in that the device provides a hand-held suturing instrument of relatively simple mechanical construction that requires no external motive source. The presently disclosed embodiments provide relative ease of operation for the surgeon with only one hand.

According to aspects illustrated herein, a suture head assembly may be removably attached to an actuator mechanism of the suturing device. The diameter of the device is small enough to fit into a typical cannula, thus making the device extremely easy to maneuver, as well as suture, during endoscopic or other MIS procedures. Also, the suture head assembly of the device can be laterally articulated to the left of center, to the right of center, up, and down, once inside the cannula, which is ideal for use in the course of endoscopic surgery, including laparoscopy, thoracoscopy and arthroscopy, as well as other less-invasive surgical procedures.

The device of the present disclosed embodiments closely emulates or replicates the manual suturing actions carried out by a surgeon. For example, during manual suturing by hand, the needle is held in forceps and travels in a circular arc with no obstructions anywhere in the interior of the arc. The design of the suturing device of the present disclosed embodiments allows for a lack of obstruction in the center of the arc of the needle during suturing. In other words, there is no hub at the center of the circular arc of the suturing needle. The entire area within the circular arc of the needle is unobstructed. This allows for the user to have better visualization during operation, unlike the present mechanical suturing methods, while maintaining control over needle movement.

A benefit provided by the suturing device of the presently disclosed embodiments is that the device enables maneuvering a suturing material through a tissue incision in a manner substantially similar to the way a surgeon would do so by hand. In particular, the suturing device first pushes a suturing needle from the tail of the needle and drives the point of the needle through the tissue. The device then picks up the point of the needle that passed through the tissue, and pulls the remainder of the suturing needle and the suture attached to the suturing needle through the tissue. The suturing needle thus consistently follows the arc of the needle's own curve, which is the preferred method of suturing, in the most atraumatic way of passing a needle through tissue. A benefit provided by the suturing device of the presently disclosed embodiments is the ability of the suturing needle to pull the suturing thread entirely through the tissue segments being closed, following each stitch. When using the suturing device of the presently disclosed embodiments, no ancillary instruments or tools such as needle holders, pick-up forceps or the like are needed to complete the stitch. A forceps can be used to tighten the knots.

According to aspects illustrated herein, there is provided a suturing device that includes a suturing needle that is protected by a housing, the suturing needle is not exposed to or handled directly by the user, thereby preventing inadvertent needle sticks. The configuration of the suturing device of the presently disclosed embodiments also protects against inadvertent penetration of internal organs or vessels by the needle, since the housing acts as a shield between the organs and the needle.

According to aspects illustrated herein, there is provided an exemplary method for suturing tissue during minimally invasive surgery that includes: (a) engaging a suturing needle with a pointed end and a second end to a suture head assembly at a distal end of a suturing device, the suture head assembly includes a curved track, whereby the suturing needle follows a curved path along the track during rotation of the suturing needle, and a latch that provides a protective housing for the suturing needle; (b) introducing the distal end of the suturing device into a body cavity; (c) positioning an opening in the needle holder assembly to span a plurality of separated tissue segments or a single tissue segment; (d) activating an actuator coupled to a drive mechanism that engages the suturing needle to cause rotational movement of the suturing needle about an axis approximately perpendicular to a longitudinal axis of the suturing device and advance the suturing needle through the plurality of separated tissue segments or the single tissue segment; (e) pulling a suturing material attached to the suturing needle through the plurality of separated tissue segments or a single tissue segment forming a stitch; and repeating steps (c) through (e) to cause a plurality of stitches to be placed through the separated tissue segments or a single tissue segment.

According to aspects illustrated herein, there is provided an exemplary method for suturing tissue during minimally invasive surgery that includes inserting a distal end of a suturing device having a suturing needle with a pointed end into a body; positioning the suturing needle to span a plurality of separated tissue segments; activating an actuator a first time causing the pointed end of the suturing needle to extend beyond a protective housing of a cartridge to engage the plurality of separated tissue segments; and activating the actuator a second time to cause the suturing needle to complete a revolution and pull a suture extending from the suturing needle through the plurality of separated tissue segments to form a stitch.

In addition to the advantages discussed above, the suturing device of the presently disclosed embodiments is relatively simple and cost efficient to manufacture. Therefore, the suturing device should find widespread suturing applications that include single stitches or continuous stitches, e.g. spiral, mattress, purse string, etc., that are required to close tissue incisions, attach grafts, or the like.

These and other advantages of the presently disclosed embodiments are illustrated through the embodiments described hereinafter. The presently disclosed embodiments accordingly comprise the features of construction, combination of elements and arrangement of parts that will be exemplified in the following detailed description.

### BRIEF DESCRIPTION OF THE DRAWINGS

The presently disclosed embodiments will be further explained with reference to the attached drawings, wherein like structures are referred to by like numerals throughout the several views. The drawings shown are not necessarily to scale, with emphasis instead generally being placed upon illustrating the principles of the presently disclosed embodiments.

**FIGS. 1-11** describe an embodiment of a device made in accordance with the invention.

**FIGS 12-25** describe still a further embodiment of a device made in accordance with the invention.

While the above-identified drawings set forth presently disclosed embodiments, other embodiments are also contemplated, as noted in the discussion. This disclosure presents illustrative embodiments by way of representation and not limitation. Numerous other modifications and embodiments can be devised by those skilled in the art which fall within the scope of the claims.

### DETAILED DESCRIPTION

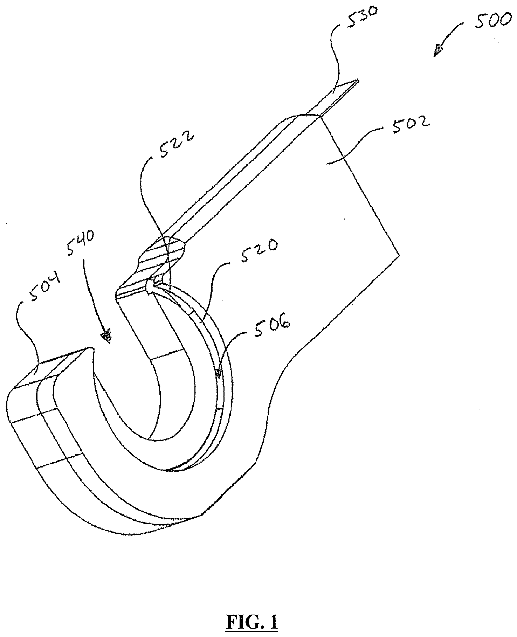

For purposes of illustration and not limitation, **FIGS. 1-11** depict an embodiment of a suturing head 500 for a suturing instrument or aspects thereof.

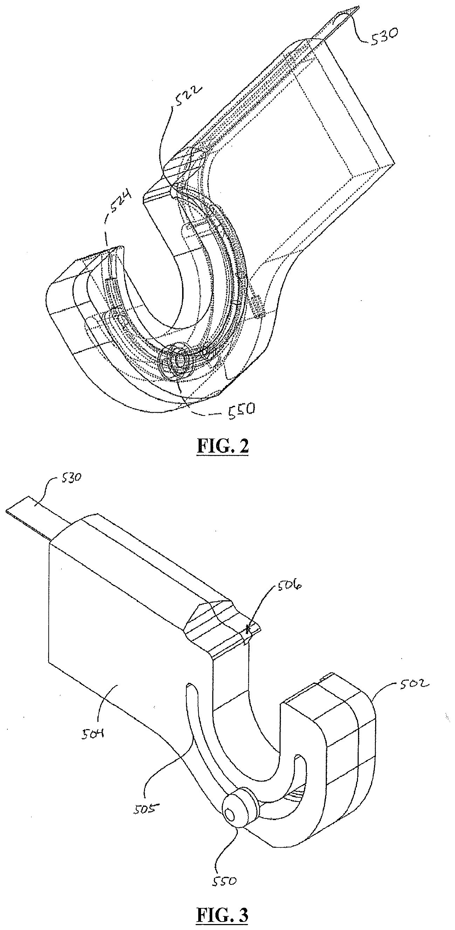

**FIGS. 1-2** illustrate perspective views of this embodiment, both in plain view and showing hidden features, respectively. As illustrated, suturing head 500 is comprised of two main housing components, 502 and 504. Housing component 502 defines a portion of a needle track 506 that is complete when components 502, 504 are assembled. This embodiment is similar to that of **FIG. 39** described in WO2008-147555, but with certain differences. Significantly, in contrast to the aforementioned embodiment of WO2008-147555, this embodiment operates by moving needle 520 through needle track 506 during a pull stroke of filament 530, rather than during a push stroke. Distal end 534 of filament 530 is attached to an engagement mechanism 550 that selectively engages notches formed in the needle 520 in a manner similar to other embodiments described herein. As depicted in **FIG. 3****,** engagement mechanism rides in an arcuate track 505 formed in housing component 504 that is generally concentric with the needle track 506. Suturing head 500 includes a tissue capture gap 540, similar to the embodiment of **FIG. 39** of WO2008-147555.

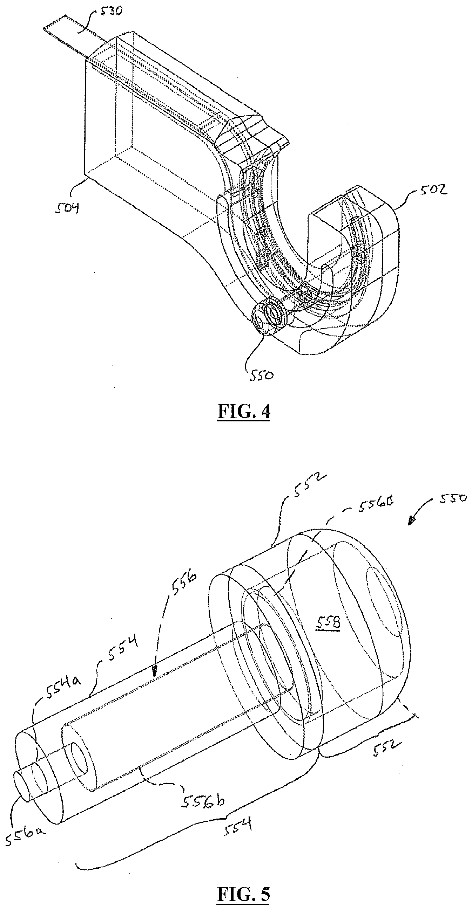

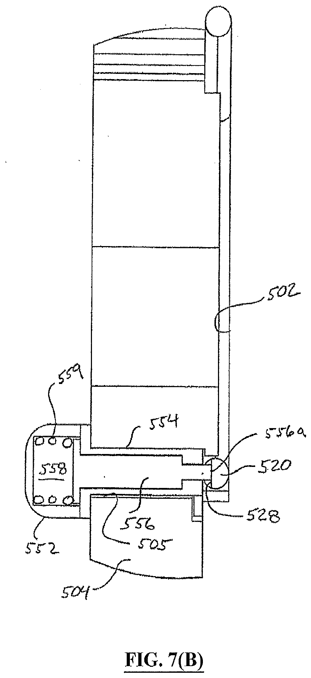

**FIG. 5** depicts the engagement mechanism that rides in track 505. As depicted, engagement mechanism 550 includes four main components: cap 552, sleeve 554, piston 556 and a chamber 558 housing a compression spring 559 (spring 559 is depicted in **FIG. 7(B)****).** In operation, sleeve 554 is affixed to distal end 534 of filament 530, piston 556 is received within sleeve 554. Next spring 559 is inserted into cap 552, which in turn is attached to sleeve 554. Reduced diameter portion 556a of piston 556 is urged through bore 554a of sleeve 554. Portion 556a of piston 556 mates with notches 526, 528 in needle 520. The operation of suturing head 500 through a complete cycle will now be described.

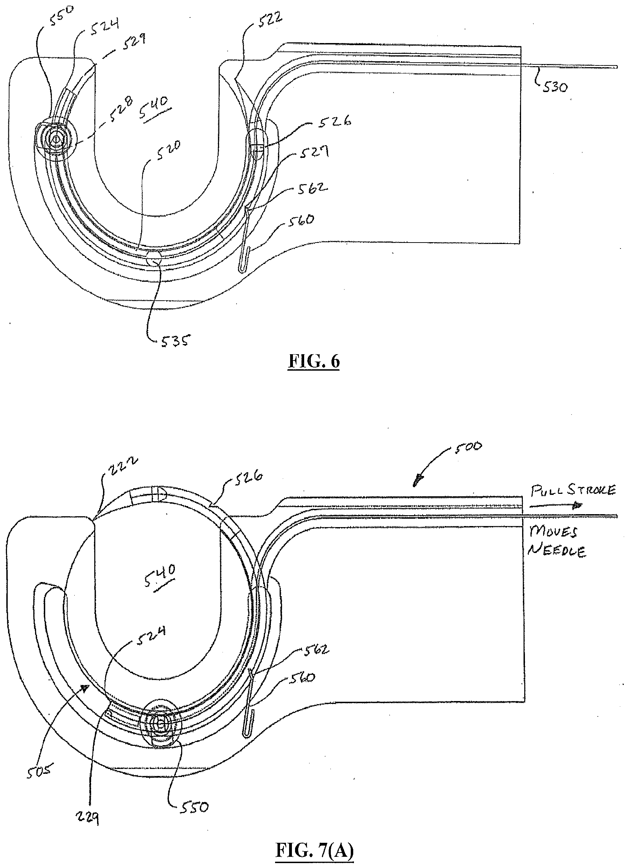

As depicted in **FIG. 6****,** needle 520 having a first pointed end 522 and a second end 524 is in the home position prior to a rotation cycle, and engagement mechanism 550 is engaged with notch 528 in needle 520. As depicted, needle includes a hollow 529 in second end 524 of needle 520 for receiving a suture (not shown). Filament 530 further includes an enlarged portion 535 for riding against needle track 506 to reduce friction and ease operation of the suturing head. As further depicted, tip 562 of pawl 560 is biased to engage with antirotate notch 527 formed into the outer circumferential surface of needle 527.

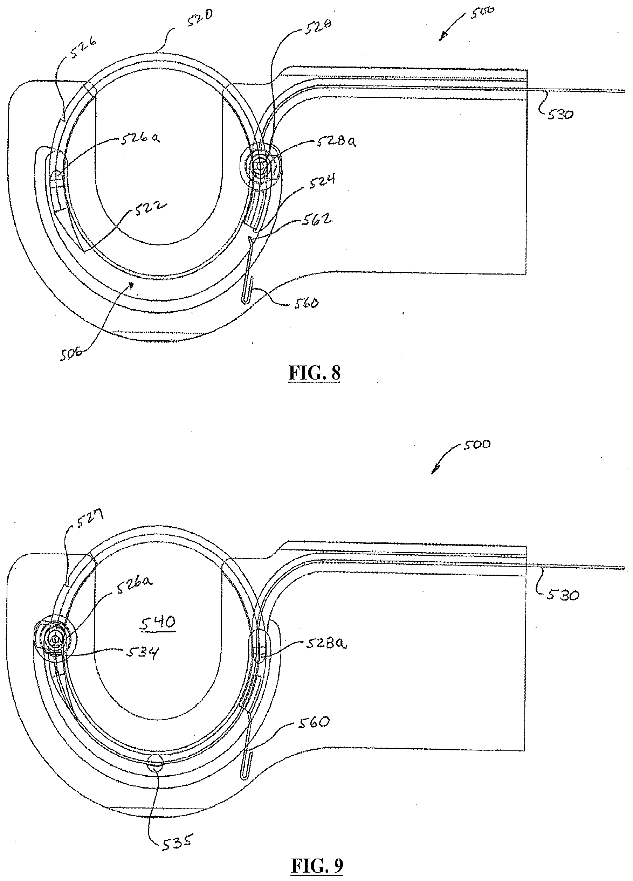

As depicted in **FIG. 7(A)****,** filament 530 is pulled proximally through the suturing instrument, causing engagement mechanism 550 to urge against notch 528 in needle, resulting in needle 520 being drawn through track 506, across gap 540, and back into track 506. Tip 562 of pawl 560 slides out of antirotate notch 527 of needle 520 and drags along the needle 520 as needle 520 moves through needle track 506. As can be seen in side cross-sectional view **FIG. 7(B)****,** end 556a of piston 556 is urged against notch 528 of needle 520 by spring 559. **FIG. 8** depicts needle 520 after having been moved through a 180 degree rotation. As can be seen in **FIG. 8****,** second end 524 of needle has moved past tip 562 of pawl 560, and tip 562 of pawl snaps into the needle track 506 to prevent the needle 520 from reversing direction. At this point, filament 530 is once again advanced distally, causing tip 556a of piston 556 to urge against the distal inclined surface 528a of notch 528. Inclined surface 528a acts as a ramp to push piston 556 into chamber 558 against the force of spring 559 until surface 556a rides up out of notch 528, and over the outer surface of needle 520 through track 505 until it passes over needle 520 and pops back out. As engagement mechanism 550 continues to be guided by arcuate notch 505, it encounters first end 522 of needle 520. The pointed end 522 of needle 520 once again acts as a ramp, compressing spring 559 as surface 556a rides up and over the needle 520 until surface 556a reaches notch 526. Upon reaching the notch 526, the piston snaps down into the notch. At this position, engagement mechanism is as depicted in **FIG. 9****.**

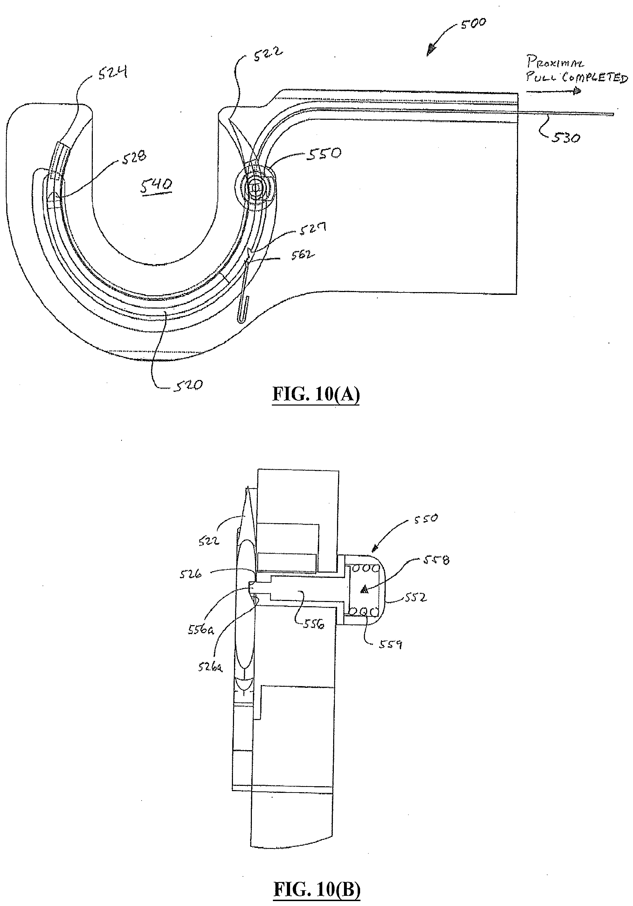

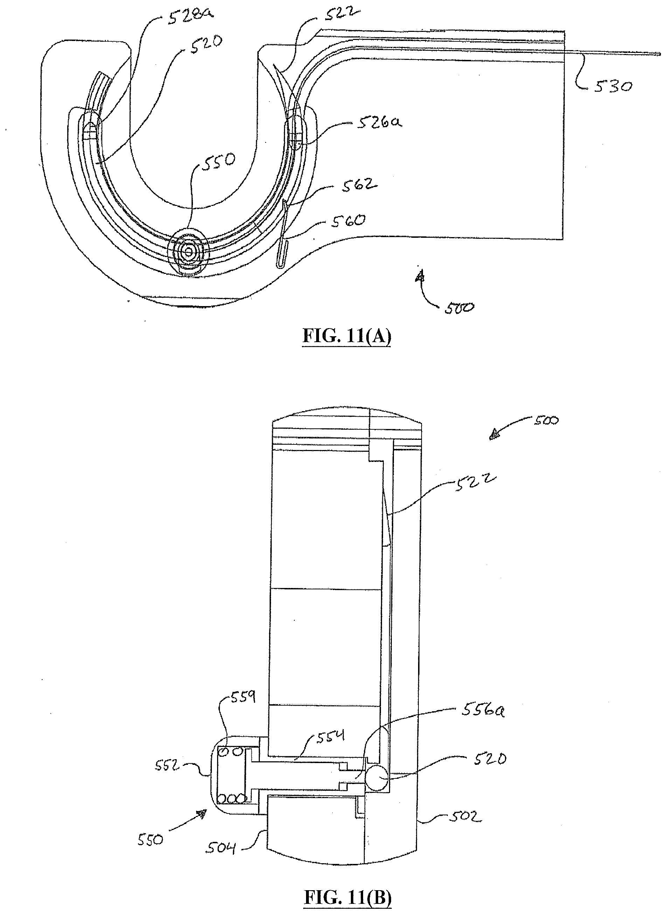

Next, filament 530 is once again pulled proximally through device causing the needle 520 to move through another 180 degree rotation, returning the needle 520 to the home position as depicted in **FIGS. 10(A)-10(B)****.** While antirotate notch 527 can move past tip 562 of pawl 560, when filament 530 is moved distally once again to pick up the needle at notch 528, needle 520 will move backward slightly until notch 527 engages with pawl tip 562. At that point, surface 556a of engagement mechanism 550 rides up inclined surface 526a and travels over the outer lateral surface of the needle 520 until the piston snaps into notch 528, preparing the suturing head 500 for another cycle as depicted in **FIGS. 11(A)-****11(B).**

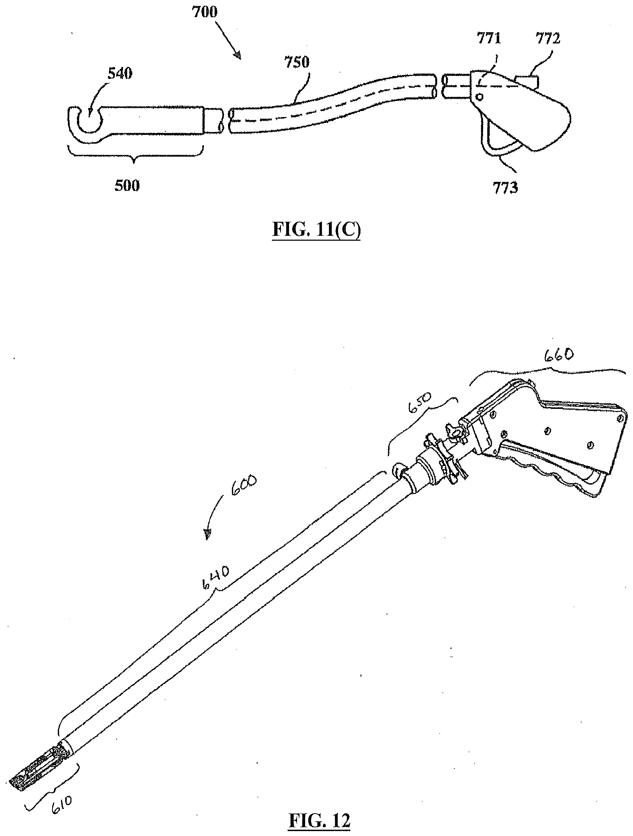

Suturing head 500 can be constructed using any desired techniques and any desired materials as described herein, for example, with reference to suturing head 356 described in WO/2008147555. Preferably, suturing head 500 is made from a polymeric material to permit manufacture of a low-cost, disposable device 700 as depicted in **FIG. 11(C)****.** Suturing head 500 can be mounted on a flexible shaft 750 as depicted in **FIG. 11(C)****,** and if desired may be mounted on an endoscope with a flexible conduit/shaft 750 arranged parallel to the endoscope, Either way, actuation and other controls (771, 772, 773) are housed within the cable/conduit 750.

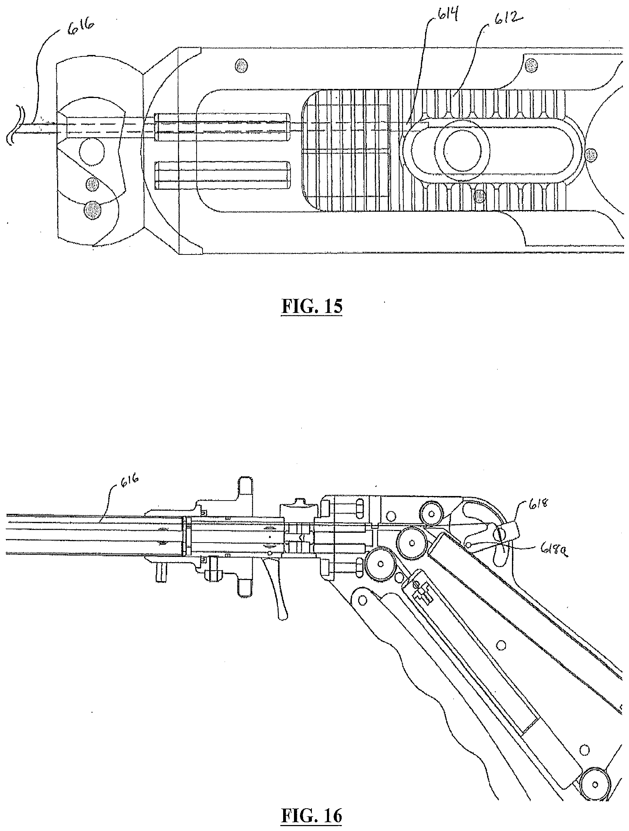

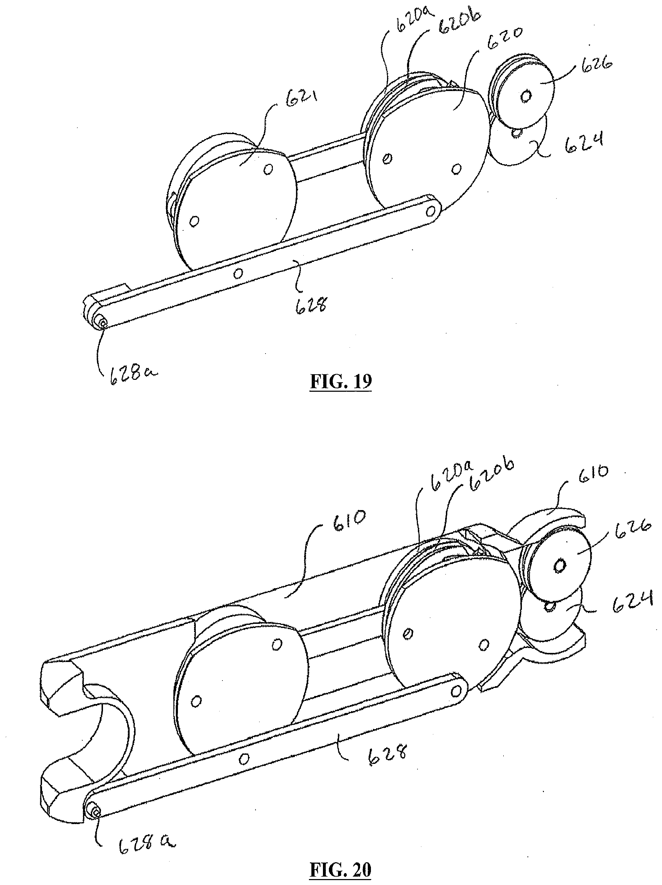

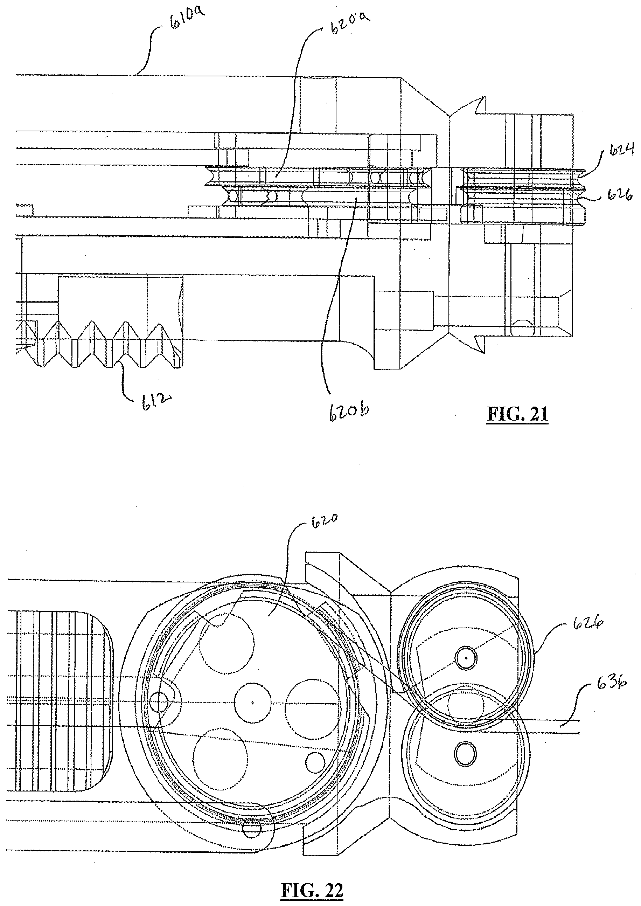

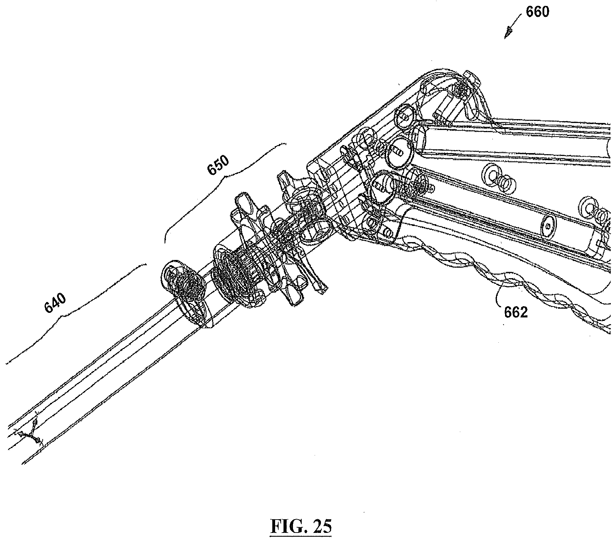

In accordance with still further aspects of the disclosure, for purposes of further illustration and not limitation, **FIGS. 12-25** depict variations of the device generally depicted at Figs. 1-38 of WO/2006034209.

As depicted in FIG. 12, device 600 is provided with a handle 660 that has been found by Applicant to be particularly user-friendly and comfortable. Device 600 also includes a suturing head 610, an elongate tubular body 640, and an roticulation region 650.

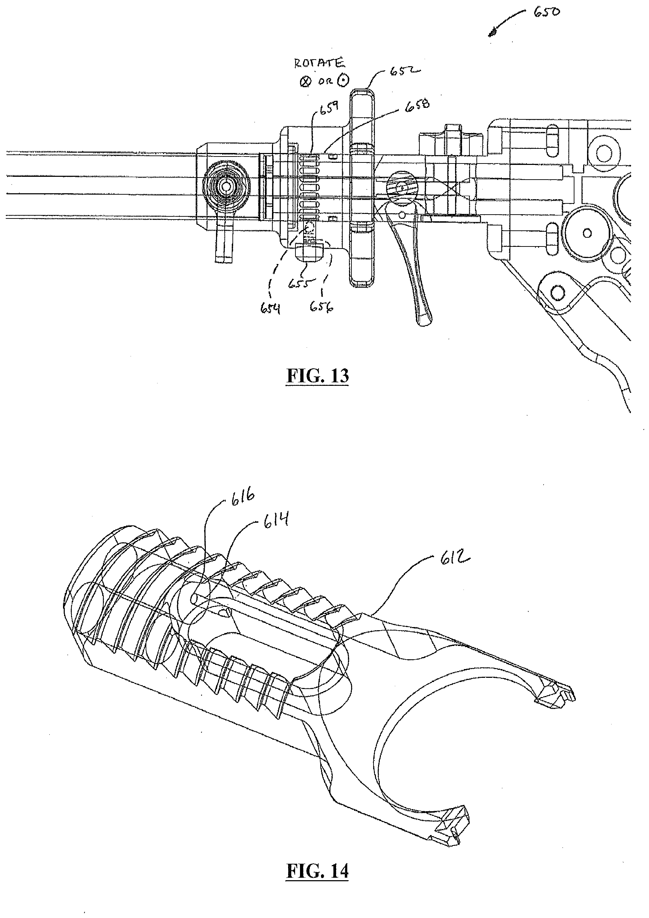

A roticulation region 650 is illustrated in **FIG. 13****.** Roticulation section 650 includes a hub 652 that is attached to tubular body 640. Hub 652 is rotatably mounted on a cylindrical bearing surface 658, having a plurality of elongate detents 659 surrounding the bearing surface 658. A detent ball 654 is contained within a detent housing 655, wherein a spring 656 urges detent ball 656 into a detent 659, preventing the hub from rotating freely, but also permitting hub to be rotated ("roticulated") about the axis of device 600, thereby permitting roticulation of the suturing head 610.

As with the suturing head depicted, for example, in **FIGS. 30-31** of WO/2006034209, a latch 612 is also provided in the embodiment of **FIG. 12** to cover the suturing needle. Specifically, as depicted in **FIGS. 14-16****,** latch 612 is provided, and is preferably biased (e.g., by a spring) to the closed position. While latch 612 can be retracted proximally by pushing on the latch 612 itself, during a procedure latch 612 may not be easily accessible. Thus, if the device 600 should jam, to avoid the difficulty in moving the latch backward to permit the needle to fall out of device, a pull wire 616 is provided that is attached at its distal end to the latch 612 (inside of a bore 614), and at its proximal end to a release trigger 618 that pivots about a point 618a. Thus, if it is desired to retract the latch 612 to permit the needle to fall out in the event of a jam, it can be released, the device 600 can be withdrawn, and the needle can be removed with forceps. The device 600 can then be reused with a new needle.

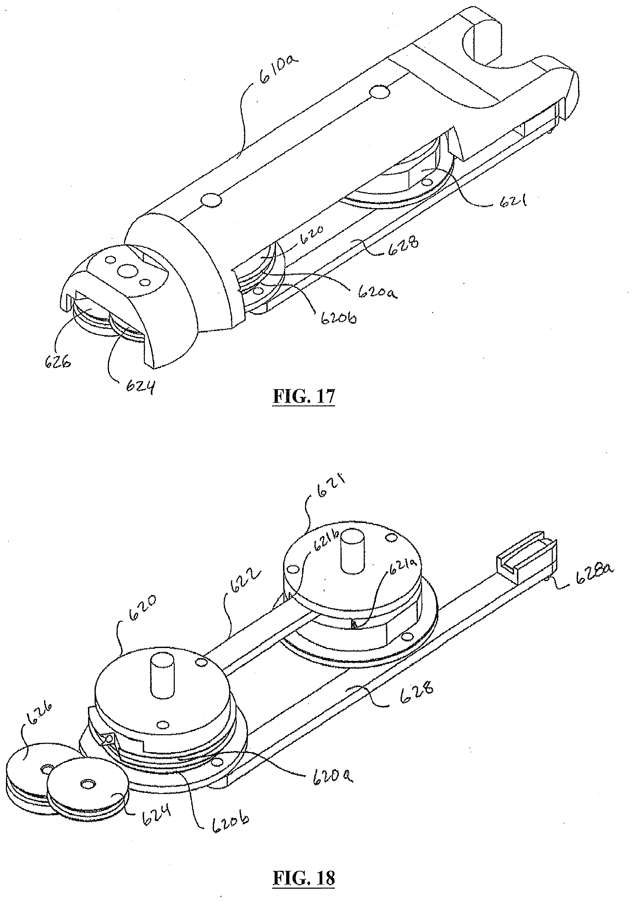

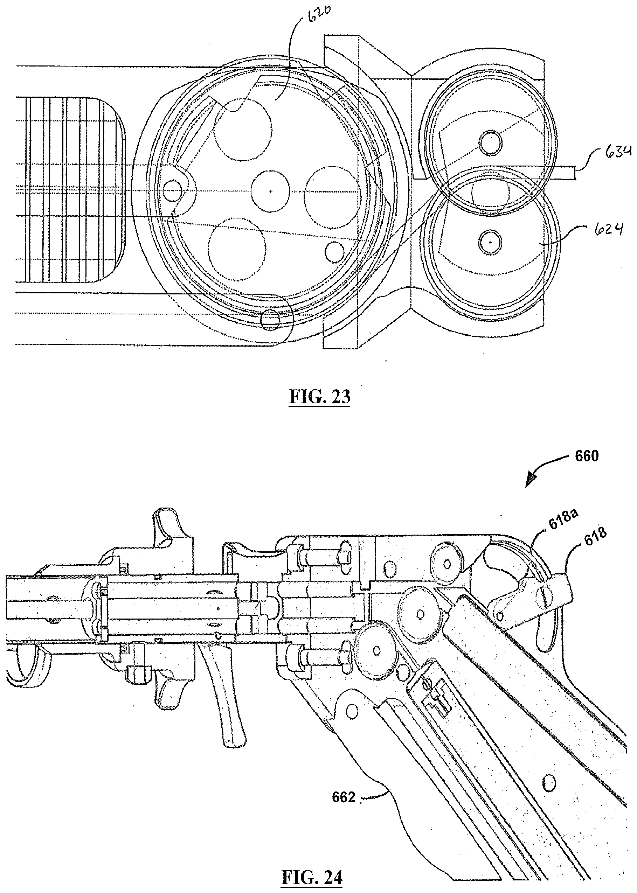

**FIGS. 17-23** depict an different drive mechanism for the suturing head 610 as compared to that depicted in WO/2006034209. Specifically, all components of the drive mechanism are fitted to one side of the suturing head 610a, rather than being anchored to both sides of the suturing head 610. This is very advantageous in assembly. Specifically, all drive components (pulleys and the like) are attached to side 610a of the suturing head. This prevents any inconvenience in needing to align the pulleys and other drive components with two opposing housing sections, and facilitates assembly generally as this design permits the drive components to be stacked and attached to a single member. As will be noted, the drive components bear some similarity to those depicted in **FIGS. 34-35** of WO/2006034209. A drive cable 634 is routed around a drive idler 624, and into the drive pulley 620a. Drive pulley 620a, in turn, drives an idler pulley 621 by way of an actuator arm 628 when advancing the suturing needle. A link or strut 622 is provided that acts as a stop for rotation of pulley 621 by engaging a bearing surface 621b in groove 621a. A needle engagement mechanism/needle assembly extension 628a is provided for driving the suturing needle (not depicted). In addition, a return cable 636 is routed around a return idler 626, and into the return pulley 620b, which is concentric with the drive pulley 620a. Return pulley 620b, in turn, drives idler pulley 621 by way of actuator arm 628 in a direction opposite from the drive pulley 620a, causing engagement mechanism 628 a to return to the home position to repeat the half cycle. **FIGS. 24-25** depict cross sectional and three dimensional wireframe views of the handle portion 660 of device 600, respectively, depicting, for example, actuator handle 662 as well as the arrangement of interior passages through which drive and return cables are routed. The return cable 636 is preferably spring-loaded so as to cause the needle engagement mechanism 628a to return to its home position.

The suturing devices of the presently disclosed embodiments can be used for laparoscopic procedures, including but not limited to laparoscopic colostomy, colectomy, adrenalectomy, splenectomy, repair of paraesophageal hernia, inguinal hernia repair, ventral hernia repair, Nissen fundoplication, liver lobectomy, gastrectomy, small bowel resection, treatment of small bowel obstruction, distal pancreatectomy, nephrectomy and gastric bypass. Those skilled in the art will recognize that the presently disclosed embodiments can be used in other laparoscopic procedures.

In using the devices of the presently disclosed embodiments, the abdomen is insufflated with gas to create a working space for the user. Any gas known to those skilled in the art including, but not limited to, nitrogen or carbon dioxide, can be used. Access portals are established using trocars in locations to suit the particular surgical procedure. A variety of surgical instruments may then be inserted into the body through these access ports/cannulas. The user then introduces the distal end portion of the suturing device into a cannula, and then articulates the suture head assembly (e.g., 500, 610). The suture head assembly is then positioned relative to the tissue/vessel to be sutured together, and the user preferably locks the suture head assembly in place. The user then, through manipulation of the suturing device, positions a plurality of separated tissue segments into the opening defined at the distal end portion of the suture head assembly. The user, using only one hand, may manipulate the device while actuating the handle to close an incision with a continuous suture whose stitches may be individually tensioned precisely and uniformly along the length of the suture similar to suturing done by hand in the conventional way. The user may employ a single suture which would extend the entire length of the incision or multiple sutures. Thus, by placement of the device spanning the incised tissue segments and actuating the handle, the suturing device enables the user to lay down a running stitch or interrupted stitch to close the tissue incision in a time efficient manner. Those skilled in the art will recognize that any conventional procedure for conducting laparoscopic surgery can be used with the device.

The minimalized structural design of the suture head assembly enables the user to have a clear, unobstructed view of the suturing needle during advancement through the tissue segments during the course of a suturing operation, thereby enabling precise placement of the suturing device to provide uniform sutures and precluding the risk of tearing tissue by placement too close to the edge of the incision. The suturing device is then advanced a short distance along the incision and the aforementioned operation is repeated to produce another stitch comprising the suturing material or thread.

The user may continue to manipulate the suturing device, alternately advancing and actuating rotation of the needle about an axis that is generally parallel to the direction of advancement to create a continuous suture which may extend through the entire length of the incision or a series of interrupted stitches. After each individual stitch is laid down, the stitch is tightened by exerting a pull on the suturing material or thread so that the resultant suture is tensioned uniformly along the length of the incised tissue segments. Therefore, a tight closure of the segments is accomplished and bleeding and tearing of tissue are minimized. Once the appropriate amount of suture material or thread 246 has been placed, the user can use a needle grasper to tighten and knot the formed stitches.

The suturing device may be configured in different ways with respect to length and angle of the suture head assembly. The size of the needle, the needle holder assembly, the aperture defined by the suturing head for receiving tissue to be sutured and the aperture position may also be varied for use in open surgery to perform procedures such as closing of the fascia, skin closure, soft tissue attachment, anastomosis, fixation of mesh, grafts and other artificial materials. Moreover, devices made in accordance with the teachings herein can be used in combination with needle loader devices described, for example, in U.S. Patent Application Serial No. 12/175,442, filed July 17, 2008.

## Claims

1. A suturing device comprising a suturing head (500), the suturing head comprising:
a) a needle track housing portion (502) including a needle (520) disposed in a needle track, the needle track being adapted and configured to guide the needle along a curved path about an axis of rotation substantially perpendicular to a longitudinal axis of the suturing head.
b) a drive housing portion containing a drive, wherein the drive includes an elongate flexible member (530), at least a portion of the elongate flexible member being adapted and configured to reciprocate along a direction substantially parallel to the longitudinal axis, the needle having:
i) a curved body, the curved body covering an arc greater than about 180°; and
ii) an engagement surface (528) for driving the needle; and **characterised by** the elongate flexible member being provided with a sleeve (554) attached to a distal end thereof, the sleeve slidably housing a drive piston (556) for engaging the engagement surface (528) of the needle, the drive piston (556) being biased against the engagement surface (528) of the needle by a compression spring (559) disposed within the sleeve (554), wherein the elongate flexible member is further adapted and configured to engage the engagement surface of the needle via the drive piston (556) and advance the needle through a circular arc as the elongate flexible member is advanced along the longitudinal axis in a proximal direction.

2. The device of Claim 1, wherein the elongate flexible member is biased to disengage from the engagement surface after advancing the needle along a portion of the needle track.

3. The device of Claim 1 or Claim 2, wherein the needle track housing portion and drive housing portion are joined along the longitudinal axis of the device.

4. The device of any preceding Claim, wherein the needle track housing portion defines a gap (540) in the needle track for receiving tissue to be sutured by the needle.

5. The device of any preceding Claim, wherein the gap is defined along a lateral edge of the needle track housing portion that is parallel to the longitudinal axis.

6. The device of any preceding Claim, wherein the elongate flexible member includes a distal end having at least one protrusion (550) for engaging with a guide track (505) that follows a path parallel to the needle track.

7. The device of any preceding Claim, wherein the elongate flexible member follows an arcuate path of a first diameter during a first portion of a drive cycle, and follows a second arcuate path of a second, different diameter during a second portion of a drive cycle.

8. The device of any preceding Claim, wherein the elongate flexible member is biased to engage with the engagement surface of the needle after advancing along an arcuate return track.

9. The device of any preceding Claim, wherein the drive is adapted to advance the needle through a 360° rotation about the axis of rotation in a plurality of drive strokes.

10. The device of Claim 9, wherein the drive is adapted to engage a first portion of the needle during a first drive stroke, and engage a second portion of the needle during a second drive stroke.

11. The device of any preceding Claim, wherein the flexible elongate member is adapted and configured to reciprocate at least in part along a curved path proximate the needle.

12. The device of any preceding Claim, wherein the engagement surface includes a notch defined by the curved body.

13. The device of any preceding Claim, wherein the engagement surface for driving the needle is defined on a surface displaced from a radially inner region of the curved body.

14. The device of any preceding Claim, wherein the drive is adapted to advance the needle through a first portion of the needle track out of the housing and into a second portion of the needle track defined in the housing.

15. The device of any preceding Claim, wherein the drive is displaced from a radially inner region of the needle.

16. The device of any preceding Claim, wherein the device defines a bite for receiving tissue, wherein the bite is substantially defined by an area circumscribed by the path of the needle.

17. The device of any preceding Claim, wherein the distal portion of the flexible elongate member follows a path that is parallel to a portion of the needle path.

18. The device of any preceding Claim, wherein the suturing head includes a pawl (560) that is adapted and configured to engage with a portion of the needle to prevent the needle from moving in a desired direction along the needle track.

## Patentansprüche

1. Nähvorrichtung, umfassend einen Nähkopf (500), wobei der Nähkopf Folgendes umfasst:
a) einen Nadelspurgehäuseabschnitt (502) einschließlich einer in einer Nadelspur angeordneten Nadel (520), wobei die Nadelspur angepasst und konfiguriert ist, um die Nadel entlang einer gekrümmten Bahn um eine Rotationsachse im Wesentlichen senkrecht zu einer Längsachse des Nähkopfs zu führen.
b) einen Antriebsgehäuseabschnitt, der einen Antrieb enthält, wobei der Antrieb ein längliches flexibles Element (530) einschließt, wobei mindestens ein Abschnitt des länglichen flexiblen Elements angepasst und konfiguriert ist, um sich entlang einer Richtung im Wesentlichen parallel zur Längsachse hin- und herzugehen, wobei die Nadel Folgendes aufweist:
i) einen gekrümmten Körper, wobei der gekrümmte Körper einen Bogen größer als ungefähr 180° abdeckt; und
ii) eine Eingriffsoberfläche (528) zum Antrieb der Nadel; und **dadurch gekennzeichnet, dass** das längliche flexible Element mit einer Hülse (554) versehen ist, die an einem distalen Ende davon angebracht ist, wobei die Hülse gleitend einen Antriebskolben (556) zum Eingriff mit der Eingriffsfläche (528) der Nadel aufnimmt; wobei der Antriebskolben (556) durch eine Druckfeder (559), die innerhalb der Hülse (554) angeordnet ist, gegen die Eingriffsfläche (528) der Nadel vorgespannt ist, wobei das längliche flexible Element weiter angepasst und konfiguriert ist, um mit der Eingriffsfläche der Nadel über den Antriebskolben (556) in Eingriff zu kommen und die Nadel durch einen kreisförmigen Bogen vorzurücken, wenn das längliche flexible Element entlang der Längsachse in einer proximalen Richtung vorgeschoben wird.

2. Vorrichtung nach Anspruch 1, wobei das längliche flexible Element vorgespannt ist, um von der Eingriffsfläche nach dem Vorrücken der Nadel entlang eines Abschnitts der Nadelspur außer Eingriff zu kommen.

3. Vorrichtung nach Anspruch 1 oder Anspruch 2, wobei der Nadelspurgehäuseabschnitt und der Antriebsgehäuseabschnitt entlang der Längsachse der Vorrichtung verbunden sind.

4. Vorrichtung nach einem der vorstehenden Ansprüche, wobei der Nadelspurgehäuseabschnitt einen Spalt (540) in der Nadelspur zur Aufnahme von Gewebe, das durch die Nadel zu nähen ist, definiert.

5. Vorrichtung nach einem der vorstehenden Ansprüche, wobei der Spalt entlang eines seitlichen Rands des Nadelspurgehäuseabschnitts definiert ist, die parallel zur Längsachse ist.

6. Vorrichtung nach einem der vorstehenden Ansprüche, wobei das längliche flexible Element ein distales Ende einschließt, das mindestens einen Vorsprung (550) zum Eingriff mit einer Führungsspur (505) aufweist, die eine Bahn parallel zur Nadelspur folgt.

7. Vorrichtung nach einem der vorstehenden Ansprüche, wobei das längliche flexible Element während eines ersten Abschnitts eines Fahrzyklus einer bogenförmigen Bahn eines ersten Durchmessers folgt und während eines zweiten Abschnitts eines Fahrzyklus einer zweiten bogenförmigen Bahn eines zweiten, anderen Durchmessers folgt.

8. Vorrichtung nach einem der vorstehenden Ansprüche, wobei das längliche flexible Element vorgespannt ist, um mit der Eingriffsoberfläche der Nadel in Eingriff zu gelangen, nachdem es sich entlang einer bogenförmigen Rückführspur bewegt hat.

9. Vorrichtung nach einem der vorstehenden Ansprüche, wobei der Antrieb angepasst ist, um die Nadel durch eine 360° -Drehung um die Drehachse in einer Vielzahl von Antriebshüben vorzuschieben.

10. Vorrichtung nach Anspruch 9, wobei der Antrieb angepasst ist, um mit einem ersten Teil der Nadel während eines ersten Antriebshubs in Eingriff zu kommen und mit einem zweiten Teil der Nadel während eines zweiten Antriebshubs in Eingriff zu kommen.

11. Vorrichtung nach einem der vorstehenden Ansprüche, wobei das flexible langgestreckte Element angepasst und konfiguriert ist, um zumindest teilweise entlang einer gekrümmten Bahn in der Nähe der Nadel hin- und herzugehen.

12. Vorrichtung nach einem der vorstehenden Ansprüche, wobei die Eingriffsoberfläche eine Kerbe einschließt, die durch den gekrümmten Körper definiert ist.

13. Vorrichtung nach einem der vorstehenden Ansprüche, wobei die Eingriffsoberfläche zum Antrieb der Nadel auf einer Oberfläche definiert ist, die von einem radial inneren Bereich des gekrümmten Körpers versetzt ist.

14. Vorrichtung nach einem der vorstehenden Ansprüche, wobei der Antrieb angepasst ist, um die Nadel durch einen ersten Abschnitt der Nadelspur aus dem Gehäuse und in einen zweiten Abschnitt der Nadelspur, der in dem Gehäuse definiert ist, vorzurücken.

15. Vorrichtung nach einem der vorstehenden Ansprüche, wobei der Antrieb von einem radial inneren Bereich der Nadel versetzt ist.

16. Vorrichtung nach einem der vorstehenden Ansprüche, wobei die Vorrichtung einen Biss zur Aufnahme von Gewebe definiert, wobei der Biss im Wesentlichen durch eine von der Bahn der Nadel umschriebene Fläche definiert ist.

17. Vorrichtung nach einem der vorstehenden Ansprüche, wobei der distale Abschnitt des flexiblen länglichen Elements einer Bahn folgt, die parallel zu einem Abschnitt der Nadelbahn ist.

18. Vorrichtung nach einem der vorstehenden Ansprüche, wobei der Nähkopf eine Sperrklinke (560) einschließt, die angepasst und konfiguriert ist, um mit einem Abschnitt der Nadel in Eingriff zu kommen, um zu verhindern, dass sich die Nadel in einer gewünschten Richtung entlang der Nadelspur bewegt.

## Revendications

1. Dispositif de suture comprenant une tête de suture (500), la tête de suture comprenant :
a) une partie boîtier de rainure d'aiguille (502) comprenant une aiguille (520) disposée dans une rainure d'aiguille, la rainure d'aiguille étant adaptée et configurée pour guider l'aiguille le long d'une trajectoire incurvée autour d'un axe de rotation sensiblement perpendiculaire à un axe longitudinal de la tête de suture,
b) une partie boîtier de mécanisme d'entraînement contenant un mécanisme d'entraînement, dans lequel le mécanisme d'entraînement comprend un élément flexible allongé (530), au moins une partie de l'élément flexible allongé étant adapté et configuré pour se déplacer selon un mouvement de va-et-vient dans une direction sensiblement parallèle à l'axe longitudinal, l'aiguille ayant :
i) un corps incurvé, le corps incurvé couvrant un arc supérieur à environ 180° ; et
ii) une surface de prise (528) pour entraîner l'aiguille; et **caractérisé en ce que** l'élément flexible allongé est doté d'un manchon (554) fixé à une extrémité distale de celui-ci, le manchon hébergeant de façon coulissante un piston d'entraînement (556) pour mettre en prise la surface de prise (528) de l'aiguille, le piston d'entraînement (556) étant sollicité contre la surface de prise (528) de l'aiguille par un ressort de compression (559) disposé à l'intérieur du manchon (554), dans lequel l'élément flexible allongé est en outre adapté et configuré pour se mettre en prise avec la surface de prise de l'aiguille via le piston d'entraînement (556) et faire avancer l'aiguille à travers un arc circulaire alors que l'élément flexible allongé est avancé le long de l'axe longitudinal dans une direction proximale.

2. Dispositif selon la revendication 1, dans lequel l'élément flexible allongé est sollicité pour se libérer de la surface de prise après avoir fait avancer l'aiguille le long d'une partie de la rainure d'aiguille.

3. Dispositif selon la revendication 1 ou la revendication 2, dans lequel la partie boîtier de rainure d'aiguille et la partie boîtier de mécanisme d'entraînement sont jointes le long de l'axe longitudinal du dispositif.

4. Dispositif selon l'une quelconque des revendications précédentes, dans lequel la partie boîtier de rainure d'aiguille définit un espace (540) dans la rainure d'aiguille pour recevoir le tissu à suturer par l'aiguille.

5. Dispositif selon l'une quelconque des revendications précédentes, dans lequel l'espace est définit le long d'un bord latéral de la partie boîtier de rainure d'aiguille qui est parallèle à l'axe longitudinal.

6. Dispositif selon l'une quelconque des revendications précédentes, dans lequel l'élément flexible allongé comprend une extrémité distale ayant au moins une protubérance (550) pour se mettre en prise avec une rainure guide (505) qui suit une trajectoire parallèle à la rainure d'aiguille.

7. Dispositif selon l'une quelconque des revendications précédentes, dans lequel l'élément flexible allongé suit une trajectoire arquée d'un premier diamètre durant une première partie d'un cycle de mécanisme d'entraînement, et suit une seconde trajectoire arquée différente, d'un second diamètre différent durant une seconde partie d'un cycle de mécanisme d'entraînement.

8. Dispositif selon l'une quelconque des revendications précédentes, dans lequel l'élément flexible allongé est sollicité pour se mettre en prise avec la surface de prise de l'aiguille après avoir progressé le long d'une rainure de retour arquée.

9. Dispositif selon l'une quelconque des revendications précédentes, dans lequel le mécanisme d'entraînement est adapté à faire avancer l'aiguille sur une rotation de 360 ° autour de l'axe de rotation en une pluralité de courses de mécanisme d'entraînement.

10. Dispositif selon la revendication 9, dans lequel le mécanisme d'entraînement est adapté pour mettre en prise une première partie de l'aiguille durant une première course du mécanisme d'entraînement, et se mettre en prise avec une seconde partie de l'aiguille durant une seconde course du mécanisme d'entraînement.

11. Dispositif selon l'une quelconque des revendications précédentes, dans lequel l'élément allongé flexible est adapté et configuré pour se déplacer selon un mouvement de va-et-vient au moins en partie le long d'une trajectoire incurvée à proximité de l'aiguille.

12. Dispositif selon l'une quelconque des revendications précédentes, dans lequel la surface de prise comprend une encoche définie par le corps incurvé.

13. Dispositif selon l'une quelconque des revendications précédentes, dans lequel la surface de prise pour entraîner l'aiguille est définie sur une surface déplacée d'une région radialement intérieure du corps incurvé.

14. Dispositif selon l'une quelconque des revendications précédentes, dans lequel le mécanisme d'entraînement est adapté à faire avancer l'aiguille à travers une première partie de la rainure d'aiguille hors du boîtier et dans une seconde partie de la rainure d'aiguille définie dans le boîtier.

15. Dispositif selon l'une quelconque des revendications précédentes, dans lequel le mécanisme d'entraînement est déplacé d'une région radialement intérieure de l'aiguille.

16. Dispositif selon l'une quelconque des revendications précédentes, dans lequel le dispositif définit une ouverture pour recevoir le tissu, dans lequel l'ouverture est sensiblement définie par une aire circonscrite par la trajectoire de l'aiguille.

17. Dispositif selon l'une quelconque des revendications précédentes, dans lequel la partie distale de l'élément allongé flexible suit une trajectoire qui est parallèle à une partie de la trajectoire d'aiguille.

18. Dispositif selon l'une quelconque des revendications précédentes, dans lequel la tête de suture comprend un cliquet (560) qui est adapté et configuré pour se mettre en prise avec une partie de l'aiguille pour empêcher l'aiguille de se déplacer dans une direction souhaitée le long de la trajectoire d'aiguille.
